# FASCICULE DE BREVET EUROPEEN

(11) **EP 0 786 988 B1**
(45) Date de publication et mention de la délivrance du brevet: **02.05.2003**
(21) Numéro de dépôt: 95935988.6
(22) Date de dépôt: 19.10.1995
(51) Int. Cl.: A61K 31/00, A61K 31/44, A61K 31/445, A61P 9/06

(54) **UTILISATION D'ANTAGONISTES DES RECEPTEURS NK1 POUR LA PREPARATION DE MEDICAMENTS A ACTION CARDIOREGULATRICE**
VERWENDUNG VON NK1-REZEPTOR ANTAGONISTEN ZUR ZUBEREITUNG VON ARZNEIMITTELN MIT KARDIOREGULIERENDER WIRKUNG
USE OF NK1 RECEPTOR ANTAGONISTS FOR PREPARING CARDIO-REGULATORY DRUGS

(30) Priorité: 21.10.1994 FR 9412589
(43) Date de publication de la demande: 06.08.1997
(73) Titulaire: SANOFI-SYNTHELABO, 75013 Paris (FR)
(72) Inventeur: GUEUDET, Christiane, F-34080 Montpellier (FR); SANTUCCI, Vincent, F-34000 Montpellier (FR); SOUBRIE, Philippe, F-34270 Saint-Mathieu-de-Tréviers (FR)
(74) Mandataire: Gillard, Marie-Louise
(86) Numéro de dépôt international: FR9501383
(87) Numéro de publication internationale: WO96012479

(56) Documents cités:
- EP-A- 0 512 901
- EP-A- 0 591 040
- IL FARMACO, vol. 48, no. 8, pages 1021-49, 'Thiolupinine and some derivatives of pharmacological interest'
- BR.J.PHARMACOL., vol. 112, no. 1, pages 250-6, 'Cardiovascular and behavioural effects of centrally administered neuropeptide K in the rat: receptor characterization'
- REGUL.PEPT., vol. 46, no. 1-2, pages 293-6, 'Role of NK-1 receptor in central cadiovascular regulation in rats: studies on a novel non-peptide antagonist, CP-96345, of substance P NK-1 receptor'
- EUR.J.PHARMACOL., vol. 222, no. 2-3, pages 213-8,

## Description

La présente invention concerne une nouvelle utilisation des antagonistes des récepteurs NK1.

Plus particulièrement, l'invention se rapporte à l'utilisation des antagonistes des récepteurs NK1 pour la préparation de médicaments à action cardiorégulatrice.

Dans la présente description on désigne par médicaments à action cardiorégulatrice les médicaments destinés à combattre les troubles de la fréquence et du rythme cardiaques, en particulier ceux qui sont occasionnés par la douleur ou le stress.

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : NK₁, NK₂, NK₃. La substance P (SP) est le ligand endogène des récepteurs NK₁, la neurokinine A (NK_{A}), celui des récepteurs NK₂ et la neurokinine B (NK_{B}), celui des récepteurs NK₃.

Les récepteurs NK₁, NK₂, NK₃ ont été mis en évidence chez différentes espèces.

Une revue récente de C.A. Maggi et al. fait le point sur les récepteurs aux tachykinines et leurs antagonistes (J. Autonomic Pharmacol., 1993, 13, 23-93).

Les antagonistes des récepteurs des neurokinines sont également décrits dans l'article de Andrew B McElroy intitulé "Neurokinine Receptor antagonists" et publié dans Patent Update 1993, p.1157-1172 ainsi que dans l'article de Russel M.Hagan et al. dans Trends Pharmacol. Sci. 1993, 14, 315-318.

Dans la description et les revendications qui suivent, les antagonistes des récepteurs NK₁, dont la substance P est le ligand endogène, sont également désignés par "NK₁-antagonistes".

Plusieurs familles d'antagonistes des récepteurs NK1 non peptidiques sont décrits dans les brevets ci-après au nom de la demanderesse :
EP-A2-428434 ; EP-A1-512901 ; EP-A1-512902, EP-A1-515240 ; EP-A1-559538 ; EP-A1-591040 ; dans les demandes françaises au nom de la demanderesse n°9403560, 9403561 et 94 03701 ainsi que dans les documents ci-après :
EP-A1-0360390 ; EP-A1-0429366 ; EP-A2-0436334 ; EP-A1-0499313 ; EP-A2-0585913 ; WO90/0552 ; WO90/05729 ; WO91/09844 ; WO91/18899 ; WO92/01688 ; WO92/06079 ; WO92/20685 ; WO 93/01170 ; WO93/21155 ; WO94/04487.

Parmi ces antagonistes spécifiques des récepteurs NK₁ on peut citer tout particulièrement les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413), RP-67580 (Mol. Pharmacol., 1994, 45 (3), 500-508) ; RP-73467 (Bioorg. Med. Chem. Lett., 1994, 4 (5), 669-672 ; RPR-100893 (Bioorg. Med. Chem. Lett., 1994 4 (5), 673-676) ; CP-99994 (Brit. J. Pharmacol., 1994, Proc. Suppl. 112, 333P)

Selon les travaux de AW DUGGAN [Neuropeptides : 1992 22, 19] on sait que la tachykinine substance P(SP) est libérée dans les cornes dorsales de la moelle en réponse à une stimulation sensorielle nociceptive. Par ailleurs les travaux de FLEETWOOD-WALKER et al. [Brain Res. 1990, 519, 169-182] ont montré que la substance P semble jouer un rôle essentiel dans la transmission des informations nociceptives au niveau central. Enfin il a été montré que la substance P était également impliquée, via les récepteurs NK1, dans le contrôle de la fonction cardiaque. A cet effet, on peut se reporter aux travaux de R.COUTURE et al. dans Naunyn-Schmiedeberg's Arch. Pharmacol. 1989 340, 547-557 et de TSCHOPE et al. dans Br.J.Pharmacol. 1992, 107, 750-755.

On a récemment montré que le SR 140333, puissant antagoniste des récepteurs NK1, bloque la réponse des neurones du thalamus à la stimulation mécanique nociceptive (EMONDS-ALT et al. Eur.J.Pharmacol.1993 250, 403-413).

Il a maintenant été trouvé que les antagonistes des récepteurs NK₁ de formule A ci-après antagonisent l'accélération habituelle du rythme cardiaque due à la douleur ou au stress. Ainsi la présente invention concerne selon un de ses aspects, l'utilisation de ces NK₁ antagonistes pour la préparation de médicaments permettant de prévenir ou de combattre les effets délétères du stress, de la douleur et leurs conséquences pathologiques, notamment au niveau cardiovasculaire ; on peut citer l'inconfort, les poussées hypertensives, l'arythmie, l'infarctus du myocarde, la mort subite.

Ainsi la présente invention concerne, selon un de ses aspects, l'utilisation pour la préparation de médicaments à action cardiorégulatrice, des antagonistes des récepteurs NK1 répondant à la formule A ci-après: 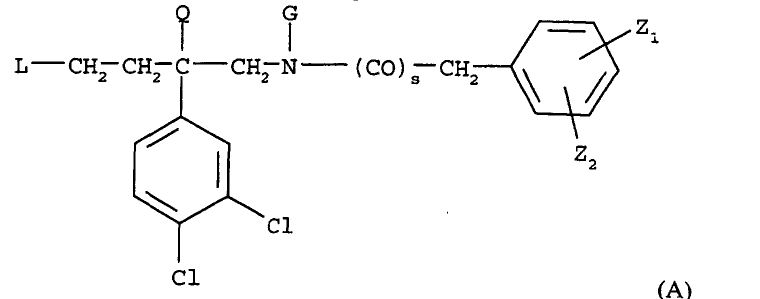 dans laquelle :
L est l'un des groupes ci-après : 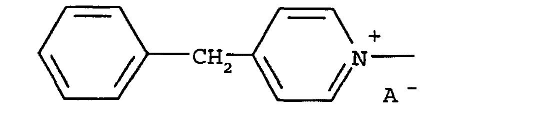 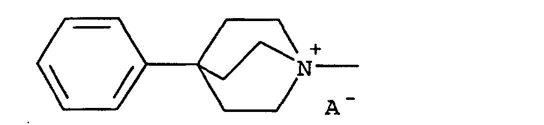 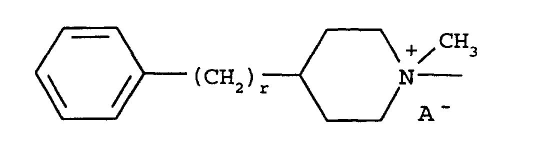 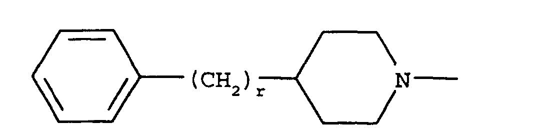
A est un anion pharmaceutiquement acceptable, de préférence un ion chlorure, méthanesulfonate, benzènesulfonate ou p-toluènesulfonate.
Q représente l'hydrogène et G représente un groupe alkyle en C₁-C₄ ou bien Q et G forment ensemble un groupe (CH₂)₃ ou CH₂CH₂CO.
Z₁ et Z₂ représentent indépendamment l'un de l'autre un halogène, un groupe (C₁-C₄)alcoxy, ou le groupe trifluorométhyle.
r, s représentent 0 ou 1, à la condition que s soit zéro uniquement lorsque Q+G est CH₂CH₂CO
et leurs sels éventuels pharmaceutiquement acceptables.

L'invention concerne également l'utilisation des énantiomères des composés définis précédemment.

Le composé particulièrement préféré aux fins de l'invention est le composé SR140333, décrit dans la demande EP-A1-0 591 040, de formule: 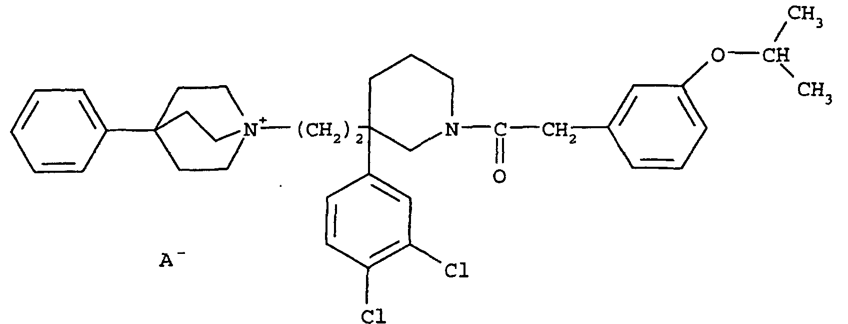 dans laquelle A⁻ est un anion pharmaceutiquement acceptable tel que par exemple chlorure, benzènesulfonate, méthanesulfonate, p-toluènesulfonate, en particulier les sels de (S)1-{2-[3-(3,4-dichlorophényl)-1-(3-isopropoxyphénylacétyl)pipéridin-3-yl]éthyl}-4-phényl-1-azoniabicyclo[2.2.2]octane qui sont le chlorure appelé SR 140333 A et le benzènesulfonate appelé SR 140333 B.

L'activité cardiorégulatrice selon la présente invention a été mise en évidence à l'aide d'un test chez le rat selon la méthode décrite dans l'exemple ci-après :

Les composés testés dans cet essai, aux doses de 0,5µg/kg i.v. provoquent une régulation du rythme cardiaque.

Les NK₁ antagonistes peuvent être utilisés pour la préparation de médicaments permettant de prévenir ou de combattre les conséquences pathologiques du stress, notamment au niveau cardiovasculaire. Les composés selon l'invention peuvent également être utilisés en association avec d'autres agents thérapeutiques agissant dans la sphère cardiovasculaire selon différents mécanismes d'action.

Grâce à la découverte de cette activité, les antagonistes des récepteurs NK1 peuvent être utilisés pour la préparation de médicaments cardiorégulateurs destinés à la normalisation de la fréquence et du rythme cardiaque. Les composés selon la présente invention, peuvent éventuellement être utilisés en association avec d'autres agents cardiorégulateurs qui agissent selon différents mécanismes d'action.

Pour leur utilisation en tant que médicaments selon l'invention, les composés antagonistes des récepteurs NK1 doivent être formulés en compositions pharmaceutiques.

Dans les compositions pharmaceutiques de la présente invention pour l'administration orale, sublinguale, sous-cutanée, intramusculaire, intraveineuse, topique, transdermique ou rectale, le principe actif peut être administré sous forme de doses unitaires, en mélange avec des supports pharmaceutiques classiques, aux animaux et aux êtres humains pour le traitement des susdites affections. Les doses unitaires appropriées comprennent les doses pour l'administration par voie orale, telles que les comprimés éventuellement sécables, les gélules, les poudres, les granules et les solutions, ou suspensions orales, les doses pour l'administration sublinguale et buccale, les doses pour l'administration sous-cutanée, intramusculaire ou intraveineuse et les doses pour l'administration rectale.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

Pour obtenir l'effet cardiorégulateur, la dose de principe actif peut varier entre 0,5 et 4000 mg par jour, de préférence entre 2,5 et 1000 mg, selon le poids et l'âge du malade et la gravité des affections à soigner:

Chaque dose unitaire peut contenir de 0,5 à 1000 mg de principe actif, de préférence de 2,5. à 250 mg ; cette dose unitaire peut être administrée 1 à 4 fois par jour.

L'invention va être maintenant illustrée plus en détail par l'exemple non-limitatif ci-après.

### EXEMPLE

La détermination de l'activité cardiorégulatrice d'un composé représentatif de l'invention (SR140333) a été effectuée selon la méthode décrite ci-dessous.

### 1.Préparation physiologique

Quinze rats mâles Sprague-Daley, d'un poids de 250-350 g (Iffa-Credo, France) ont été anesthésiés à l'uréthane (1,25 g/kg i.p.) et placés en contention stéréotaxique. La veine latérale de la queue a été cathétérisée afin de permettre l'injection du composé à tester ou de leur véhicule (eau distillée + 3 gouttes de Tween 80, 1 ml/kg i.v.). L'enregistrement de la fréquence cardiaque a été effectué au moyen de deux électrodes métalliques insérées dans le tissus sous-cutané de part et d'autre de la cage thoracique. Le signal a été amplifié, filtré et transformé grâce à un discriminateur à fenêtre en impulsions TTL (Transistor Transistor Logic) selon le standard défini par "International Electrotechnical Commission" et reconnu aux Etats Unis par Electronic Industry Association. C'est un signal binaire (logic 0 : 0 volt, logic 1 : 5 volts). Ces impulsions ont été traitées en temps réel sur PC pour fournir des histogrammes de rythme en impulsions/min. On a mesuré en temps différé sur ces histogrammes les paramètres adéquats.

### 2. Procédure expérimentale

On a enregistré le rythme cardiaque spontané et on l'a laissé se stabiliser pendant 15 à 25 min. avant le début des stimulations mécaniques. Celles-ci ont consisté à pincer une patte postérieure au moyen d'une pince à pression calibrée (P≈ 50 psi) pendant une durée de 3s. Deux à 4 pincements ont été effectués à intervalles de 5 min. avant l'administration intraveineuse du produit SR 140333 A ou de son véhicule. A partir du moment de l'injection, les stimulations ont été appliquées toutes les 10 minutes sur une période maximum de 85 minutes.

### 3. Traitement des données

On a mesuré, pour chaque stimulation, le nombre d'impulsions cardiaques pendant la période d'une minute précédant, et d'une minute suivant le début de la stimulation. Dans la période pré-injection, on a déterminé la variation du nombre d'impulsions ainsi obtenues à chacune des stimulations et on l'a comparée à celle des périodes précédant les stimulations ; on a calculé ensuite la moyenne des valeurs ainsi obtenues. Dans la période post-injection, on a seulement déterminé la variation du rythme par rapport à son niveau antérieur à la stimulation. On a comparé cette variation pour les divers temps à partir de l'injection à la moyenne des variations de la période pré-injection.

### 4. Résultats

Les résultats sont résumés sur le tableau I. La stimulation mécanique nociceptive par pincement de la patte postérieure provoquait une élévation modérée mais significative du rythme cardiaque qui excédait les 3 secondes de la stimulation proprement dite. Lorsque le véhicule était injecté seul il n'y avait pas de différence de cette élévation pour les stimulations successives. L'injection du SR 140333 A (0.5µg/kg i.v.) a provoqué une abolition quasi totale de la réponse cardiaque à la stimulation. Cette diminution restait statistiquement significative pendant plus d'une heure après l'injection, temps au cours duquel on notait une certaine récupération de la réponse. Les pourcentages d'inhibition étaient successivement de 97, 96, 99, 96, 80, 66 et 48 pour les temps 5, 15, 25, 35, 45, 55 et 65 minutes post-injection, respectivement.

### 5. Conclusions

Les résultats de la présente étude montrent que l'antagoniste sélectif des récepteurs NK1 SR 140333 A bloque l'accélération du rythme cardiaque provoquée par la stimulation mécanique nociceptive.

**TABLEAU I**

| Effets du SR140333 A sur la réponse cardiaque à la stimulation nociceptive pour le temps 5 minutes après administration. Variation induite par la stimulation ^{a} | | | | |
|---|---|---|---|---|
| | Rythme de base ^{a} | pré-injection | 5 minutes post-injection | % inhibition ^{d} |
| véhicule seul^{b} | 503 ± 39 | +35 ± 9* | +37 ± 10* | -6 |
| SR140333 A bc | 517 ± 35 | +67 ± 11* | +2 ± 1 Υ | 97 |

| | | | | |
|---|---|---|---|---|
| a en battements/minute | | | | |
| b les valeurs indiquent la moyenne ± ESM, n = 5 rats | | | | |
| c 0.5 µg/kg i.v. | | | | |
| d post-pré-injection | | | | |
| * augmentation significative (P < 0.05) / rythme de base | | | | |
| Υ diminution significative (P < 0.05) / valeur pré-injection statistiques : ANOVA + test de Dunnett | | | | |

## Revendications

1. Utilisation d'un antagoniste des récepteurs NK1 pour la préparation de médicaments à action cardiorégulatrice, ledit antagoniste étant un composé de formule : 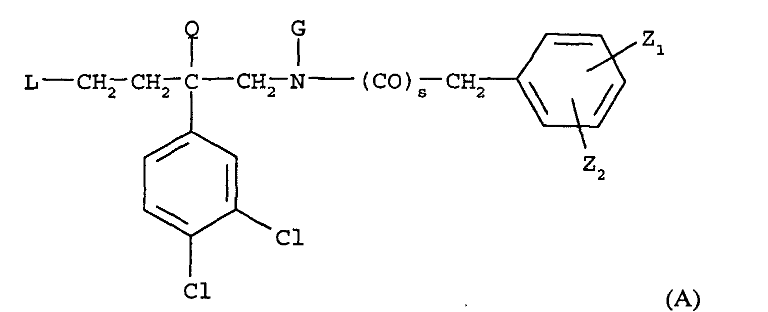 dans laquelle :
L est l'un des groupes ci-après : 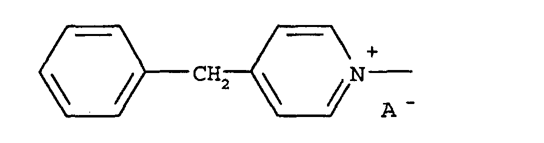 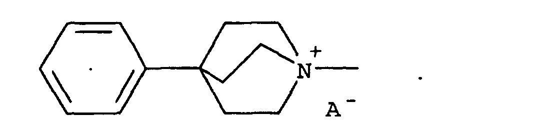 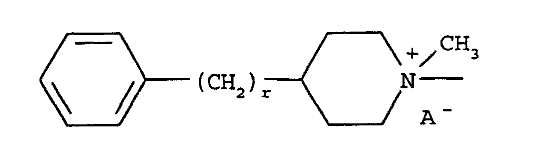 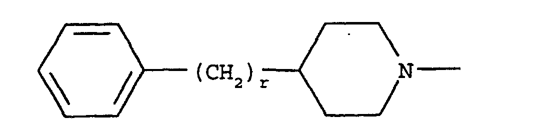
A est un anion pharmaceutiquement acceptable, de préférence un ion chlorure, méthanesulfonate, benzènesulfonate ou p-toluènesulfonate ;
Q représente l'hydrogène et G représente un groupe alkyle en C₁-C₄ ou bien Q et G forment ensemble un groupe (CH₂)₃ ou CH₂CH₂CO ;
Z₁ et Z₂ représentent indépendamment l'un de l'autre un halogène, un groupe (C₁-C₄)alcoxy, ou le groupe trifluorométhyle ;
r, s représentent 0 ou 1, à la condition que s soit zéro uniquement lorsque Q+G est CH₂CH₂CO ;
ses énantiomères et ses sels éventuels pharmaceutiquement acceptables.

2. Utilisation d'un antagoniste des récepteurs NK1 pour la préparation de médicaments à action cardiorégulatrice, ledit antagoniste étant le composé de formule : 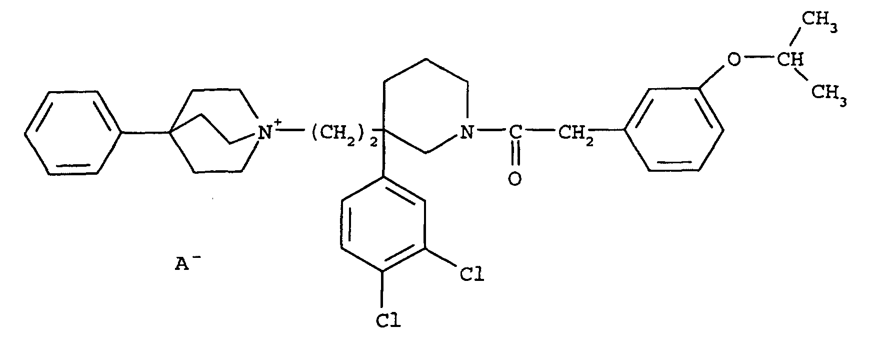 dans laquelle A⁻ est un anion pharmaceutiquement acceptable tel que chlorure, benzènesulfonate, méthanesulfonate et p-toluènesulfonate.

3. Utilisation selon la revendication 2, **caractérisé en ce que** l'antagoniste du récepteur NK1 est le chlorure ou le benzènesulfonate de (S) 1-{2-[3-(3,4-dichlorophényl)-1-(3-isopropoxyphénylacétyl)pipéridin-3-yl]éthyl}-4-phényl-1-azoniabicyclo[2.2.2]octane.

4. Utilisation selon l'une quelconque des revendications 1 à 3 pour la préparation de médicaments utiles pour prévenir ou combattre les troubles liés au stress.

5. Utilisation selon l'une quelconque des revendications 1 à 3 pour la préparation de médicaments utiles pour prévenir ou combattre les troubles liés à la douleur.

6. Utilisation selon l'une quelconque des revendications 1 à 3 pour la préparation de médicaments utiles pour antagoniser l'accélération du rythme cardiaque provoquée par la douleur ou le stress.

## Patentansprüche

1. Verwendung eines Antagonisten der NK1-Rezeptoren zur Herstellung von Medikamenten mit kardioregulierender Wirkung, wobei der Antagonist eine Verbindung der Formel 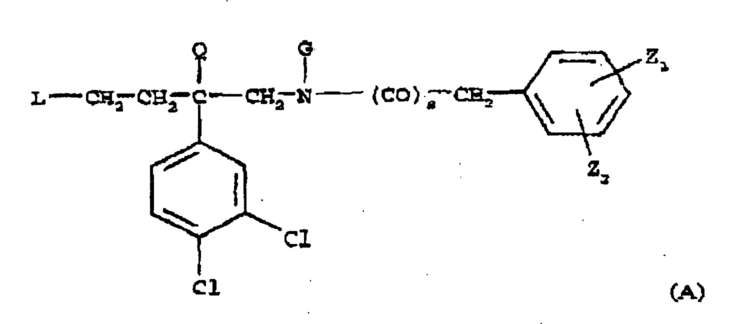 worin:
L eine der nachstehenden Gruppen ist: 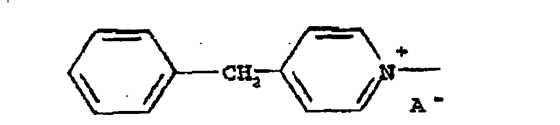 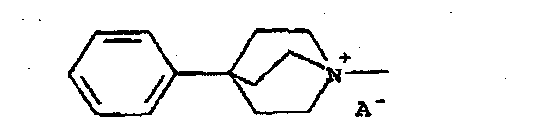 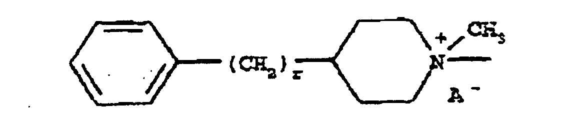 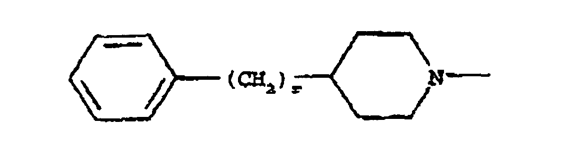
A ein pharmazeutisch annehmbares Anion, vorzugsweise ein Chlorion, Methansulfonat, Benzolsulfonat oder p-Toluolsulfonat ist;
Q Wasserstoff bedeutet und G eine (C₁-C₄)-Alkylgrupppe darstellt, oder aber Q und G zusammen eine Gruppe (CH₂)₃ oder CH₂CH₂CO bilden;
Z₁ und Z₂ unabhängig voneinander ein Halogen, eine (C₁-C₄)-Alkoxygrupppe oder Trifluormethyl darstellen;
r, s für 0 oder 1 steht, mit der Maßgabe, dass s nur dann Null ist, wenn Q+G für CH₂CH₂CO steht;
Enantiomere derselben oder mögliche pharmazeutisch annehmbare Salze derselben.

2. Verwendung eines Antagonisten der NK1-Rezeptoren zur Herstellung von Medikamenten mit kardioregulierender Wirkung, wobei der Antagonist eine Verbindung der Formel 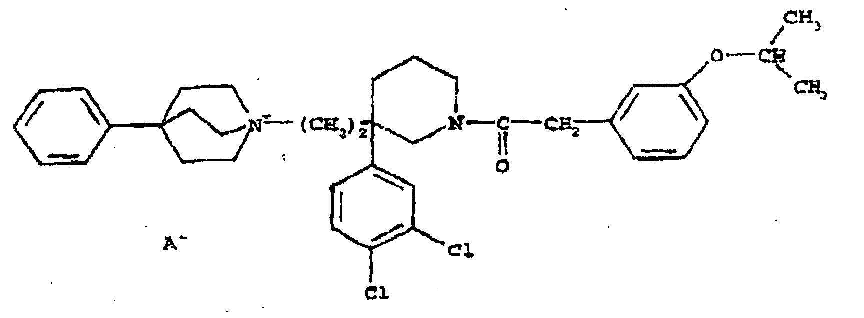 ist, worin A⁻ ein pharmazeutisch annehmbares Anion wie Chlor, Benzolsulfonat, Methansulfonat oder p-Toluolsulfonat ist.

3. Verwendung nach Anspruch 2, **dadurch gekennzeichnet, dass** der Antagonist des NK1-Rezeptors (S) 1-{2-[3-(3,4-Dichlorphenyl)-1-(3-isopropoxyphenylacetyl)piperidin-3-yl]ethyl}-4-phenyl-1-azoniabicyclo[2.2.2]octanchlorid oder -benzosulfonat ist.

4. Verwendung nach einem der Ansprüche 1 bis 3 zur Herstellung von Medikamenten, die zur Prävention oder Bekämpfung von durch Stress bedingten Beschwerden nützlich sind.

5. Verwendung nach einem der Ansprüche 1 bis 3 zur Herstellung von Medikamenten, die zur Prävention oder Bekämpfung von durch Schmerz bedingten Beschwerden nützlich sind.

6. Verwendung nach einem der Ansprüche 1 bis 3 zur Herstellung von Medikamenten, die zur Antagonisierung von durch Schmerz oder Stress verursachter Herzrhythmusbeschleunigung nützlich sind.

## Claims

1. Use of an NK₁ receptor antagonist for the preparation of drugs with a cardioregulatory action, said antagonist being a compound of the formula: 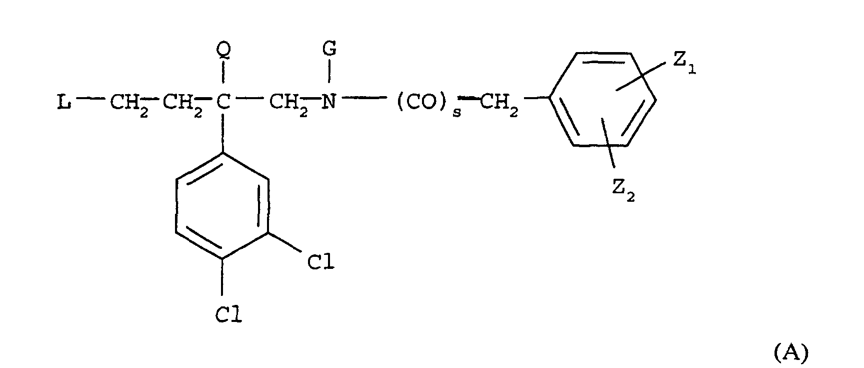 in which:
L is one of the following groups: 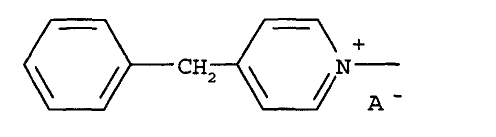 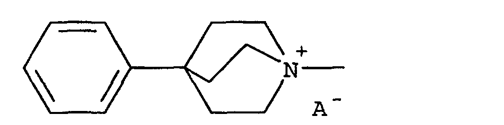 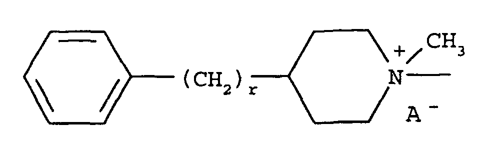 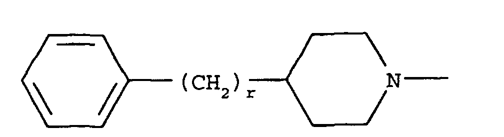
A is a pharmaceutically acceptable anion, preferably a chloride, methanesulfonate, benzenesulfonate or p-toluenesulfonate ion;
Q is hydrogen and G is a C₁-C₄-alkyl group, or Q and G together form a group (CH₂)₃ or CH₂CH₂CO;
Z₁ and Z₂ independently of one another are a halogen, a (C₁-C₄)alkoxy group or the trifluoromethyl group; and
r and s are 0 or 1, with the proviso that s is zero only when Q + G is CH₂CH₂CO;
its enantiomers and its possible pharmaceutically acceptable salts.

2. Use of an NK₁ receptor antagonist for the preparation of drugs with a cardioregulatory action, said antagonist being a compound of the formula : 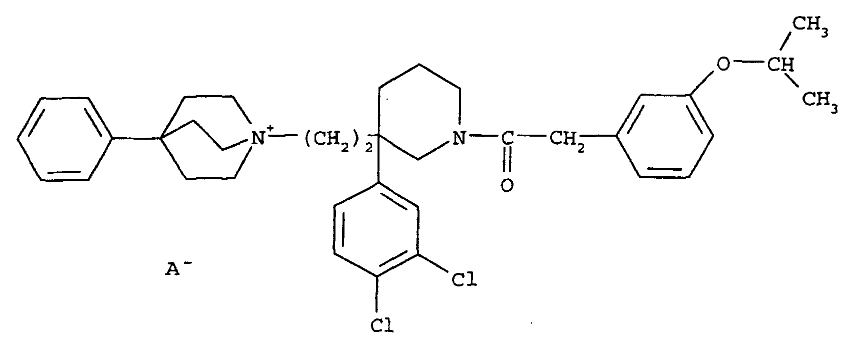 in which A⁻ is a pharmaceutically acceptable anion such as chloride, benzenesulfonate, methanesulfonate and p-toluenesulfonate.

3. Use according to claim 2, **characterized in that** the NK₁ receptor antagonist is the chloride or benzenesulfonate of (S)-1-{2-[3-(3,4-dichlorophenyl)-1-(3-isopropoxyphenylacetyl)piperidin-3-yl]ethyl}-4-phenyl-1-azoniabicyclo[2.2.2]-octane.

4. Use according to anyone of claims 1 to 3 for the preparation of drugs which are useful for preventing or controlling stress-related disorders.

5. Use according to anyone of claims 1 to3 for the preparation of drugs which are useful for preventing or controlling pain-related disorders.

6. Use according to anyone of claims 1 to 3 for the preparation of drugs which are useful for antagonizing the acceleration of the heart rhythm which is caused by pain or stress.
